(19) Europäisches Patentamt
European Patent Office
Office européen des brevets

(11) **EP 1 151 272 B1**

(12) **EUROPEAN PATENT SPECIFICATION**

(45) Date of publication and mention of the grant of the patent:
**30.09.2009 Bulletin 2009/40**

(51) Int Cl.:
*G01N 15/08* (2006.01)   *G01N 27/00* (2006.01)
*G01N 27/26* (2006.01)   *G01N 27/12* (2006.01)

(21) Application number: **99960357.4**

(22) Date of filing: **15.11.1999**

(86) International application number:
**PCT/US1999/027047**

(87) International publication number:
**WO 2000/029829 (25.05.2000 Gazette 2000/21)**

(54) **SIMULTANEOUS DETERMINATION OF EQUILIBRIUM AND KINETIC PROPERTIES**

GLEICHZEITIGE BESTIMMUNG VON GLEICHGEWICHTS- UND KINETISCHEN EIGENSCHAFTEN

DETERMINATION SIMULTANEE DES PROPRIETES CINETIQUES ET D'EQUILIBRE

(84) Designated Contracting States:
**AT BE CH CY DE DK ES FI FR GB GR IE IT LI LU MC NL PT SE**

(30) Priority: **16.11.1998 US 108674 P**
**17.11.1998 US 108915 P**

(43) Date of publication of application:
**07.11.2001 Bulletin 2001/45**

(60) Divisional application:
**08016749.7 / 2 096 427**

(73) Proprietor: **CALIFORNIA INSTITUTE OF TECHNOLOGY**
**Pasadena, CA 91125 (US)**

(72) Inventors:
• **LEWIS, Nathan, S.**
**La Canada, CA 91011 (US)**
• **SEVERIN, Erik, J.**
**San Marino, CA 91108 (US)**
• **FREUND, Michael**
**Altadina, CA 91001 (US)**
• **MATZGER, Adam, J.**
**Pasadena, CA 91106 (US)**

(74) Representative: **Bizley, Richard Edward et al**
**HLBBshaw**
**Merlin House**
**Falconry Court**
**Baker's Lane**
**Epping, Essex CM16 5DQ (GB)**

(56) References cited:
**US-A- 5 571 401**      **US-A- 5 591 898**

• STUSSI E ET AL: "Chemoresistive conducting polymer-based odour sensors: Influence of thickness changes on their sensing properties" SENSORS AND ACTUATORS B, ELSEVIER SEQUOIA S.A., LAUSANNE, CH, vol. 43, no. 1-3, 1 September 1997 (1997-09-01), pages 180-185, XP004103396 ISSN: 0925-4005
• K. BODENHÖFER, A. HIERLEMANN, G. NOETZEL, U. WEIMAR AND W. GÖPEL: "Performances of mass-sensitive devices for gas sensing: thickness shear mode and surface acoustic wave transducers" ANAL. CHEM., vol. 68, no. 13, 1 July 1996 (1996-07-01), pages 2210-2218, XP002204258
• YADONG JIANG ET AL: "Preparation and properties of organic polymer sub-micrometer function films" ELECTRETS, 1996. (ISE 9)., 9TH INTERNATIONAL SYMPOSIUM ON SHANGHAI, CHINA 25-30 SEPT. 1996, NEW YORK, NY, USA, IEEE, US, 25 September 1996 (1996-09-25), pages 678-683, XP010212635 ISBN: 0-7803-2695-4
• BODENHOFER K ET AL: "Chiral discrimination by simple gas sensors" 1997 INTERNATIONAL CONFERENCE ON SOLID-STATE SENSORS AND ACTUATORS. DIGEST OF TECHNICAL PAPERS. TRANSDUCERS 97. CHICAGO, IL, JUNE 16 - 19, 1997. SESSIONS 3A1 - 4D3. PAPERS NO. 3A1.01 - 4D3.14P, INTERNATIONAL CONFERENCE ON SOLID-STATE SENSORS AND ACTU, vol. 2, 16 June 1997 (1997-06-16), pages 1391-1394, XP010240744 ISBN: 0-7803-3829-4

**Description**

**CROSS-REFERENCES TO RELATED APPLICATIONS**

The present application claims priority to U.S. Provisional Patent Application No. 60/108,674, filed November 16, 1998, and U.S. Provisional Patent Application No. 60/108,915, filed November 17, 1998.

**STATEMENT AS TO RIGHTS TO INVENTIONS MADE UNDER**

**FEDERALLY SPONSORED RESEARCH AND DEVELOPMENT**

The research embodied in the present application was supported in part by Grant No. DAAK60-97-K-9503 awarded by DARPA and Grant No. DAAG55-97-1-0187 awarded by the United States Army. The government may have rights in any patent issuing on this application.

**BACKGROUND OF THE INVENTION**

Polymer-based chemical sensors exist in a large variety of forms and types, differing in application, sensitivity, operating principles, types of response and stability. Conducting polymer devices are popular and operate under the principle of a change in response upon exposure to different volatiles. A wide variety of polymers are used for such devices including substituted pyrroles and anilines.

Such organic polymer-based sensors have found use in a variety of different applications and devices including, for example, devices that function as analogs of the mammalian olfactory system (see, U.S. Patent No. 5,571,401, which issued to Lewis et al., Lundström et al., Nature 352:47-50 (1991) and Shurmer and Gardner, Sens. Actuators B 8:1-11 (1992)), bulk conducting polymer films (Barker et al., Sens. Actuators B 17:143 (1994) and Gardner et al., Sens. Actuators B 18:240 (1994)), surface acoustic wave devices (Grate et al., Anal. Chem. 67:2162 (1995), Grate et al., Anal. Chem. 65:A987 (1993) and Grate et al., Anal. Chem. 65:A940 (1993)), fiber optic micromirrors (Hughes et al., J. Biochem. and Biotechnol. 41:77 (1993)), quartz crystal microbalances (Chang et al., Anal. Chim. Acta 249:323 (1991)) and dye impregnated polymeric coatings on optical fibers (Walt et al., Anal. Chem. 68:2191 (1996)).

Studies have shown that conducting polymers are sensitive to a wide range of gases and vapors and can be used in gas-sensing microelectronic devices. In certain instances, a basic model for a polymer gas sensor consists of a thin uniform polymer film lying on top of a pair of coplanar electrodes supported by an insulating substrate. It is assumed that the gas, or vapor, diffuses into the film and is adsorbed at sites randomly distributed throughout the film. The diffusion and adsorption equations can be presented in terms of several fundamental dimensionless parameters which describe the underlying chemical and physical properties of the system. (see, Gardner et al., IEE Proc., Circuits Devices Syst. 142, 321-33, (1995)).

The concept of an electronic nose that mimics the mammalian olfactory sense is now well established. An essential feature of an electronic nose is an array of chemical sensors such as polymer based sensors, coupled with computerized multivariate statistical processing tools. These sensors respond to a wide variety of analytes giving rise to a unique signature or pattern for a given analyte. The pattern is interpreted using pattern recognition algorithms to identify or quantify the analyte of interest. Using pattern recognition algorithms, the output signature, such as an electrical response, can be correlated and compared to the known output signature of a particular analyte or mixture of substances. By comparing the unknown signature with the stored or known signatures, the analyte can be detected, identified and quantified. Examples of such detection devices can be found in U.S. Patent Numbers 5,571,401, which issued to Lewis et al.; 5,675,070, which issued to Gelperin; 5,697,326, which is issued Mottram et al.; 5,788,833 which issued to Lewis et al.; 5,807,701, which issued to Payne et al.; and 5,891,398, which issued to Lewis et al..

Using the combination of an array of non-specific gas sensors and suitable pattern recognition techniques, it is possible to build electronic systems that mimic the ability of the mammalian olfactory system for the discrimination and classification of odors. Potential applications for such systems include quality control in the food and beverage industries, as well as environmental monitoring and mammalian health. Recently, the availability of first generation commercial instrumentation has stimulated considerable interest in sensor development.

The need to have arrays of sensor elements is necessary because the power of discrimination increases with the diversity of the type of sensors in the array. The variation or differentiation in the sensor array can be achieved in various fashions. For instance, in bulk conducting polymer sensors, a different polymer can be used in the fabrication of each sensor element. Using this fabrication technique, a 32 sensor array would requires 32 different polymers.

What is needed in the art is a polymer based sensor system that shows intra-array variation without necessarily changing the polymer itself. Such a system would allow simultaneous determination of kinetic and equilibrium properties of an analyte. The present invention fulfills these and other needs.

## SUMMARY OF THE INVENTION

The discrimination of sensor arrays increases with the number of sensors in the array. In certain polymer based sensors, it is desirable to have intra-array variation while maintaining the polymer constant or by using relatively fewer polymers.

As such, the present invention provides a sensor array according to claim 1.

Using the sensor arrays of the present invention it is possible to detect and identifying various analytes. Moreover, using the sensor arrays of the present invention it is possible to determine both equilibrium or steady-state parameters as well as kinetic or diffusion coefficient information. In certain instances, the steady-state data can be used for analyte determination whereas the kinetic data can be used for sensor array optimization and design.

In another aspect, the present invention provides a method for measuring a diffusion coefficient of an analyte, according to claim 7.

Moreover, in certain instances, the present invention provides a method for simultaneously determining a partition coefficient and a diffusion coefficient according to claim 8.

These and other embodiments will become more apparent when read with the detailed description and accompanying drawings that follow.

## BRIEF DESCRIPTION OF THE DRAWINGS

Figure 1 illustrates a response of a sensor array of the present invention.
Figure 2 illustrates various responses as a function of sensor thickness and analyte volatility.

## DETAILED DESCRIPTION OF THE INVENTION AND PREFERRED EMBODIMENTS

## I. SENSORS

The present invention provides a sensor device comprising a sensor array for detecting an analyte in a fluid and a detector capable of detecting an electrical response operatively associated with said sensor array comprising: a first sensor having a first predetermined polymer thickness and a second sensor having a second predetermined polymer thickness wherein the first predetermined polymer thickness is different than the second predetermined polymer thickness wherein the polymer of the first and second sensor is the same and has a different thickness, and wherein the thickness of the first sensor is comprised between 0.01 $\mu$m and 20 $\mu$m. The polymer can be a conductive polymers with conductive fillers; conductive polymers with conductive fillers and an insultaing polymer.

As used herein the term "predetermined" means that the polymer layer associated with the sensor has a predefined or predetermined thickness which is measured within about 1 nm using atomic force microscopy, scanning tunneling microscopy, profilometry or other suitable method known by those of skill in the art.

In a preferred embodiment, the sensors of the present invention are disclosed in U.S. Patent No. 5,571,401. Briefly, the sensors described therein are conducting materials and nonconducting materials arranged in a matrix of conducting and nonconducting regions. The nonconductive material can be a nonconducting polymer such as polystyrene. The conductive material can be a conducting polymer, carbon black, an inorganic conductor and the like. The sensor arrays comprise at least two sensors, typically about 32 sensors and in certain instances 1000 or more sensors. The array of sensors can be formed on an integrated circuit using semiconductor technology methods, an example of which is disclosed in PCT Patent Application Serial No. WO 99/08105, entitled "Techniques and Systems for Analyte Detection," published February 19, 1999. Another preferred sensor is disclosed in WO 99/27357 entitled "Materials, Method and Apparatus for Detection and Monitoring Chemical Species," published June 3, 1999.

Preferably, the sensor array of the present invention comprise sensors selected from the following group of sensors: organic conducting polymers such as polymers of pyrrole and aniline, or insulating polymers with conductive fillers and nonconducting/conducting regions sensors.

In one embodiment, the sensors are those described in U.S. Patent No. 5,945,060, issued August 31, 1999 to Buehler. As described therein, the sensor or resistor element has a plurality of different electrode configurations. These electrode configurations include: U-bend, comb and serpentine shapes. The resistor elements are then exposed to predetermined known gases. Patterns of characteristic changes in the conductance of the resistor elements in response to exposure to the predetermined gases is then measured and stored.

As further described in the patent issued to Buehler, a gas sensor can be fabricated on a substrate that includes a plurality of electrodes wherein each electrode has a plurality of shapes or dimensions. Polymer films are deposited over the electrodes. The polymer films have a conductivity that changes when the film is exposed to various gases as measured across the electrodes. As used in the present invention, the polymer films have varying predetermined thicknesses to generate an array of sensors. Thereafter, a current measuring circuit is connected to the electrodes to measure conductivity changes. An optimal geometric configuration for the electrode is selected based on prior tests of the film's response to various gases of interest. By having a electrode geometry as well as other factors that optimizes the polymer film's response to a particular gas, the gas sensor exhibits an improved ability to distinguish among different gases. The sensor permits the optimization of numerous other factors such as polymerization process by control of the polymerization heat using the heater elements, polymer film doping and other factors.

The sensor devices, methods and systems of the present invention can detect a wide range of differential electrical responses.

## II. POLYMERS

The sensors of the present invention have a predetermined polymer thickness. In the presence of an analyte, a sensor comprising a predetermined polymer thickness, will sorb the analyte, and produce a detectable response. A detector operatively associated with the sensor detects the response. In certain embodiments, the polymer is nonconductive and is mixed with an electrically conductive material. In other instances, the sensor comprises nonconductive regions of organic polymer, such as insulating organic polymer, interspersed with regions of electrically conductive material. In still other instances, the polymer is a conducting polymer, an insulating polymer with conductive fillers or a plasticized polymer. As the polymer of the sensor sorbs an analyte, the properties of the sensor changes.

Many different polymers can be used. Suitable insulating organic polymers include, but are not limited to, main-chain carbon polymers such as poly(dienes), poly(alkenes), poly(acrylics), poly(methacrylics), poly(vinyl ethers), poly(vinyl thioethers), poly(vinyl alcohols), poly(vinyl ketones), poly(vinyl halides), poly(vinyl nitriles), poly(vinyl esters), poly(styrenes), poly(arylenes), and the like, main-chain acrylic heteroatom organic polymers such as poly(oxides), poly(carbonates), polyesters), poly(anhydrides), poly(urethanes), poly(sulfonates), poly(siloxanes), poly(sulfides), poly(thioesters), poly(sulfones), poly(sulfonamides), poly(amides), poly(ureas), poly(phosphazenes), poly(silanes), poly(silazanes), and the like, and main-chain heterocyclic polymers such as poly(furan tetracarboxylic acid diimides), poly(benzoxazoles), poly(oxadiazoles), poly(benzothiazinaphenothiazines), poly(benzothiazoics), poly(pyrazinoquinoxalines), poly(pyromellitimides), poly(quinoxalines), poly(benzimidazoles), poly(oxindoles), poly(oxoisoindolines), poly(dioxoisoindolines), poly(triazines), poly(pyridazines), poly(piperazines), poly(pyridines), poly(piperidines), poly(triazoles), poly(pyrazoles), poly(pyrrolidines), poly(carboranes), poly(oxabicyclononanes), poly(dibenzofarans), poly(phthalides), poly(acetals), poly(anhydrides), carbohydrates, and the like. In a preferred embodiment, the polymers employed are poly(vinyl acetate) (PVA) and poly (methacrylate) (PMMA) and polystyrene (PS). Each of the above organic polymers, and the monomer units that polymerize to form these polymers, are well known to those of skill in the art.

One or more of a variety of electrically conductive materials can be employed as the electrically conductive material. In some embodiments, the conductive material is a conducting polymer, or an insulating polymer with conductive fillers. Moreover, in certain other embodiments, the conductive material is a metal such as gold, silver, platinum or other conductive metals well known to those of skill in the art. In other embodiments, the conductive material is a carbonaceous material such as carbon blacks, graphite, coke, $C_{60}$, and the like. In a preferred embodiment, the conductive material is carbon black. In an equally preferred embodiment, the conductive material can be a particle, such as a gold nanoparticle with a capping ligand shell (*see,* WO 99/27357).

In certain embodiments, the polymers of the present invention comprise a compatible molecule of low volatility (a plasticizer) as disclosed in U.S. Serial Number 09/338,339, filed June 22, 1999. In another embodiment, the organic mixture comprises a plasticizer combined with an organic polymer blend of a first organic polymer and a second organic polymer. In certain embodiments, the first or second polymers are both insulating polymers. In still another embodiment, the organic mixture comprises a plasticizer combined with an organic polymer formed from a first organic monomer and a second monomer. In yet another embodiment, the organic mixture comprises a plasticizer combined with an interpenetrating network comprising a first organic polymer and a second organic polymer formed from an organic monomer polymerized in the presence of the first organic polymer.

In another embodiment, the present invention provide polymer based sensors comprising the plasticized polymeric electrolytes (PPE) (*see,* K. Buhlmann et al., Sensors and Actuators B, 49, 156-165 (1998). As disclosed therein, the PPE comprises three components: a polymer; a plasticizer; and an organic salt. The polymers included polyvinylchloride (PVC), celluloseacetate, (CA), poly(epichlorohydrin) (PECH), poly(ethyleneoxide) (POX), poly(vinylpropionate) (PVP), poly(-isobutylene) (PIB) from Sigma/Aldrich GmbH (Germany) and polyurethane from Elastogran BASF (Germany).

Suitable plasticizers include, but are not limited to, bis-(2-ethylhexyl)phthalate (BEHP), bis-(2-ethylheyxl)sebacate (DOS), dibutylsebacate (DBS), 2-fluorophenyl-2'-nitro-phenylether (2F2NE), *ortho*-nitrophenyloctylether (*o*-NPOE), tris-(2-ethylhexyl)phosphate (TEHP), tris-(2-ethylhexyl)trimelliate (TEHT) were from Fluka. Triphenylphosphate (TPP) was from Sigma/Aldrich. Organic salts used included potassiumtetrakis-(4-chlorophenyl)borate (KTpCPB), tridodecylmethylammoniumchloride (TD-MAC), tridodecylmethylammoniumnitrate (TDMAN-03), tetradodecylammonium-(4-chlorophenyl)borate (TDApCPB) and tetraoctylammoniumbromide (TOAB) were all from Fluka.

WO 99/00663, published January 7, 1999, discloses a combinatorial approach for preparing arrays of chemically sensitive polymer-based sensors which are capable of detecting the presence of a chemical analyte in a fluid contact therewith. The described methods and devices comprise combining varying ratios of at least first and second organic materials which, when combined , form a polymer or polymer blend that id capable of absorbing a chemical analyte, thereby providing a detectable response. The detectable response of the sensors prepared by this method is not linearly related to the mole fraction of at least one of the polymer-based components of the sensor.

In still yet another embodiment, the conductive region can be a conductive particle, such as a colloidal nanoparticle. In this embodiment, the term "polymer" means nanoparticles comprising a central core conducting element and an insulating attached ligand optionally in a polymer matrix. These metallic nanoparticles can be synthesized using a variety of methods. In a preferred method of synthesis, a modification of the protocol developed by Brust *et al.* can be used. (*see,* Brust, M.; Walker, M.; Bethell, D.; Schiffrin, D.J.; Whyman, R. J. Chem. Soc., Chem. Commun., 1994, 801-802.) As used herein the term "nanoparticle" refers to a conductive cluster, such as a metal cluster, having a diameter on the nanometer scale. Examples of colloidal nanoparticles for use in accordance with the present invention are described in the literature (*see,* Templeton et al. J. Am. Chem. Soc. (1998) 120 :1906-1911; Lee et al., Isr. J. Chem. (1997) 37: 213-223 (1997); Hostetler et al. LANGMUIR (1998) 14:17-30; Ingram et al., J. Am. Chem. Soc., (1997) 119 : 9175-9178; Hostetler et al., J. Am Chem. Soc. (1996) 118 :4212-4213; Henglein J. Phys. Chem. (1993) 97 :5457-5471; Zeiri, J. Phys. Chem.(1992) 96 :5908-5917; Leff et al., LANGMUIR (1996) 4723-4730. Another preferred nanoparticle is disclosed in WO 99/27357 entitled "Materials, Method and Apparatus for Detection and Monitoring Chemical Species," published June 3, 1999.

In another preferred embodiment, the polymers are disclosed in WO 99/31494, which published on June 24, 1999. As disclosed therein, the sensors comprise substituted polythiophenes. One preferred embodiment is poly (3,3"-dihexyl-2-2":S',2"-terthiophene).

### III. ANALYTE DETECTION AND APPLICATIONS

A wide variety of analytes can be detected using the devices, methods and systems of the present invention. Suitable analytes include, but are not limited to, alkanes, alkenes, alkynes, dienes, alicyclic hydrocarbons, arenes, alcohols, ethers, ketones, aldehydes, carbonyls, carbanions, heterocycles, polynuclear aromatics, organic derivatives, biomolecules, off-gasses of microorganisms, microbes, bacteria and viruses, sugars, nucleic acids, isoprenes, isoprenoids, fatty acids and their derivatives.

Moreover, in certain embodiments, the methods and systems of the present invention can be used for monitoring medical conditions and disease processes. For instance, WO 98/29563, published July 9, 1998. discloses a method for monitoring conditions in a patient wherein a sample is obtained from a patient over a period of time. The samples are then flowed over a gas sensor and a response is measured. Thereafter, the response is correlated with known responses for known conditions. The conditions include, but are not limited to, the progression and or regression of a disease state, bacterial infections, viral, fungal or parasitic infections, the effectiveness of a course of treatment and the progress of a healing process.

In another embodiment, the methods and systems of the present invention can be used for monitoring medical conditions in a respiring subject. For instance, WO 98/39470, published September 11, 1998 discloses a method for detecting the occurrence of a condition in a respiring subject. The method comprises introducing emitted respiratory gases to a gas sensing device, detecting certain species present in the gas and correlating the presence of the species with certain conditions.

A wide variety of conditions can be ascertained using this aspect of the present invention. These conditions include, but are not limited to, halitosis, ketosis, yeast infections, gastrointestinal infections, diabetes, alcohol, phenylketonuria, pneumonia, and lung infections. Those of skill in the art will know of other conditions and diseases amenable to the methods and systems of the present invention.

In yet another embodiment, the methods and systems of the present invention can be used for monitoring conditions and disease processes in female patients. For instance, WO 99/09407, published February 25, 1999 discloses a method for detecting the occurrence of a condition in a female patient comprising obtaining a sample of gaseous or volatile substance from the vaginal region of the patient, detecting the gas and correlating the detection with the occurrence of a condition.

A wide variety of conditions can be ascertained using this aspect of the present invention. These conditions include, but are not limited to, cervical cancer, ovarian or uterine cancer, HIV, sexually transmitted diseases, cytomegalovirus, yeast infections, pregnancy and Chlamydia.

In still yet another embodiment, the methods and systems of the present invention can be used for monitoring conditions and disease processes using a device that affixes to a portion of the skin on a subject. For instance, WO 99/09408, published February 25, 1999 discloses a method for detecting a condition of a subject with a device that is adapted to be affixed to the subject and having a gas sensing means disposed so as to detect gases and volatile species emanating from a portion of the skin.

A wide variety of conditions can be ascertained using this aspect of the present invention. These conditions include, but are not limited to, skin cancer, diabetes, heart disease, heavy metal in the subject and drugs.

In addition to the foregoing applications, a wide variety of commercial applications are available for the sensors arrays and electronic noses including, but not limited to, environmental toxicology and remediation, biomedicine, materials quality control, food and agricultural products monitoring, heavy industrial manufacturing, ambient air monitoring, worker protection, emissions control, product quality testing, oil/gas petrochemical applications, combustible gas detection, $H_2S$ monitoring, hazardous leak detection and identification, emergency response and law enforcement applications, illegal substance detection and identification, arson investigation, hazardous spill identification, enclosed space surveying, explosives detection, utility and power applications, emissions monitoring, transformer fault detection, food/beverage/agriculture applications, freshness detection, fruit ripening control, fermentation process monitoring and control applications, flavor composition and identification, product quality and identification, refrigerant and fumigant detection, cosmetic/perfume, fragrance formulation, product quality testing, patent protection applications, personal identification, chemical/plastics/pharmaceutical applications, fugitive emission identification, leak detection, solvent recovery effectiveness, perimeter monitoring, product quality testing, hazardous waste site applications, fugitive emission detection and identification, leak detection and identification, perimeter monitoring, transportation, hazardous spill monitoring, refueling operations, shipping container inspection, diesel/gasoline/aviation fuel identification, building/residential natural gas detection, formaldehyde detection, smoke detection, automatic ventilation control applications (cooking, smoking, etc.), air intake monitoring, hospital/medical anesthesia & sterilization gas detection, infectious disease detection and breath applications, body fluids analysis, pharmaceutical applications, drug discovery and telesurgery.

In one embodiment, the sensor arrays of the present invention are used to evaluate the progress of chemical reactions, such as asymmetrical synthesis, in a quantitative or qualitative fashion. In this aspect, the arrays provide quick assays for example, the enantiomeric excess or purity of compound, such as in a combinatorial library of catalysts forming the compounds of interest. The methods and devices described herein allow detection and selection of promising leads in a rapid fashion from multiwell plates, and also allow for evaluation of certain compounds for chiral similarity and enantiomeric purity.

## IV. SENSOR FABRICATION

Methods of sensor fabrication are numerous. A method for fabricating an array of sensors for detecting an analyte in a fluid comprises: depositing a polymer layer onto a first sensor forming a first predetermined polymer thickness; depositing a polymer onto a second sensor having a second predetermined polymer thickness wherein the first predetermined polymer thickness is different than the second predetermined polymer thickness.

The sensors of the present invention can be fabricated using a spin casting process. In certain aspects, the spin casting process involves use of a thin-film material dissolved in a volatile liquid solvent. The solution is poured on the sample and the sample is rotated at high speed. Centrifiagal force spreads the material and after the solvent evaporates, a thin layer of film remains on the sample. In this aspect, the thickness depends on the solubility of the deposited material and the spin film, and typically is in the range from about 0.01 $\mu$m to about 20 $\mu$m, and preferably between about 0.05 $\mu$m to about 2 $\mu$m. Using spin casting, it is possible to fabricate provide an array of sensor elements with various predetermined thicknesses.

The sensors of the present invention can be fabricated using sputtering techniques. Sputtering is performed in a vacuum chamber. After evacuation of air, an inert gas, such as argon or helium, is introduced into the chamber at about $2X10^{-6}$ to $5X10^{-6}$ Torr. An external high voltage power supply is attached to the cathode which is fabricated of the material to be deposited on the sample. The sample is attached to the anode at some distance from the cathode. High voltage ignites a plasma of the inert gas and the gas ions bombard the target. The kinetic energy of the bombarding ions is sufficiently high to free some atoms from the target surface. Hence, the escaped sputtered atoms deposit on the surface of the sample. Using this technique, very uniform polymer layers are possible. Moreover, materials from more than one target can be deposited at the same time (co-sputtering) permitting a controlled ratio of materials.

In another aspect, a chemical vapor phase deposition process is used for the fabrication of sensor array having varying predetermined thicknesses. In this technique, the substrates are positioned on a stationary or rotating table and thereafter the temperature is elevated to the required level by means of the heating elements. The chamber lid has an inlet for the carrier $H_2$ gas, that can be added by various precursors and dopants. These additives, while being carried over the heated surface of the substrate, form a polymer film layer. Using this technique, sensor arrays can be fabricated having varying predetermined thicknesses.

In another aspect, sensors of the present invention can be coated with a thin layer of chemoselective material providing highly sensitive chemical sensors for the detection and monitoring of vapors and gases using a thin film deposition technique such as matrix assisted pulsed laser evaporation (MAPLE) and MAPLE-Direct Write (MAPLE-DW). In a preferred aspect, the MAPLE technique is used to coat high quality polymer films on SAW devices, and conventional pulsed laser deposition is used to deposit a passivation layer of diamond-like-carbon on a SAW device surface to prevent water adsorption. (*see,* R. Andrew McGill, et al., IEEE Transactions on Ultrasonics, Ferroelectrics, and Frequency Control 45:1370-1380 (1998)). Those skilled in the art will know of other deposition techniques suitable for use in fabricating sensors of the present invention.

In certain other aspects, the fabrication techniques of the present invention makes use of a common hobbyist's air-brush designed for decorative art. In certain aspects, a polymer solution or suspension is placed in a reservoir and this solution or suspension is stirred while the application is occurring. The apparatus is attached to a regulated supply or compressed air. The flowing air allows the solution or suspension to be drawn from the reservoir and is then ejected from the nozzle of the air brush. This flow of polymer, solvent, and, if present, suspended material is directed toward substrates that have been prepared to receive the films and thereafter become sensor elements.

Advantageously, this air-brush method is amenable to automation. Many sensor elements can be fabricated in the time it takes to make one using some prior art methods and has the reproducibility of the best previous methods. A number of well known robotic systems have also been developed and can be adapted for use in the present invention. These systems include automated workstations like the automated apparatus developed by Takeda Chemical Industries, LTD. (Osaka, Japan) and many robotic systems utilizing robotic arms (Zymate II, Zymark Corporation, Hopkinton, Mass.; Orca, Hewlett-Packard, Palo Alto, Calif.) which mimic the manual synthetic operations performed by a person of skill in the art. The nature and implementation of modifications to these devices so that they can operate as discussed herein will be apparent to skilled artisans.

Moreover, the air-brush method allows various and odd shaped substrates to be employed. The substrates do not need to be flat and without interruptions in the flat surface. The substrates can be set up such that a single pass of the airbrush coats a large number of sensors. This leads to a large number of very reproducible sensors. The reproducibility can be measured using the baseline resistance difference (BRD). The BRDs between sensors using the airbrush method were all within about 5% of each other. This technology can also be applied to small jets for the purpose of depositing into small wells or onto specific regions on an otherwise flat substrate. In certain aspects, the spray can be made to pass over the whole substrate surface or be made to be directed at only a small area. Custom jet sprayers can be made to direct conical sprays, linear sprays, planer sprays, or other spray geometries.

## V. EQUILIBRIUM CONSTANTS AND DIFFUSION COEFFICIENTS

In another embodiment, the present invention provides systems, methods and devices for simultaneously determining an equilibrium constant (*i.e.,* partition coefficient) as well as a diffusion coefficient. In certain instances, the thinner polymer layers are used to determine the partition coefficient and rapid identification of the analyte, whereas the thicker polymer layers can then be used to determine a diffusion coefficient.

The partition coefficient, K, is defined as $K = C_s/C_v$, wherein $C_s$ is the concentration of analyte (solute) in the sorbent phase and $C_v$ is the concentration of the analyte in the vapor phase at equilibrium (at steady-state). In the experimental protocol used herein, $C_v$ is constant since the vapor generation apparatus is continuously replenishing the vapor stream.

Using the sensors of the present invention, it is possible to concurrently or subsequently determine the diffusion coefficient of an analyte with respect to a particular polymer. As such, the present invention provides a method for measuring a diffusion coefficient of an analyte, comprising: contacting a first sensor having a first predetermined polymer thickness to elicit a first response; contacting a second sensor having a second predetermined polymer thickness to elicit a second response; comparing the first response to the second response to calculate a time lag and thereafter measuring the diffusion coefficient of the analyte.

In this aspect, it is assumed that the analyte and the polymer membrane or layer do not react. In the absence of a reaction between the analyte and the polymer, Flick's law applies *i.e.,*

$$\frac{\partial C}{\partial t} = D\frac{\partial^2 C}{\partial x^2}$$

$$\text{Equation I}$$

wherein C is the concentration of the analyte and D is the diffusivity. In order to measure the diffusivity experimentally in a polymer based sensor, the time lag procedure is used. Using this procedure, a plot of flux ($\Delta(\Delta R/R_i)/\Delta t$) versus times yields a straight line whose intercept θ *i.e.,* the time lag, on the t axis is represented by Equation II.

$$D = \frac{L^2}{6\theta}$$

<div align="right">Equation II</div>

From this graphical relationship the time lag $\theta$ is determined. $\Delta R$ is equal to $R_t$-$R_i$ wherein $R_t$ is the resistance at time t and $R_i$ is the initial resistance. L is the predetermined polymer thickness of the sensor, the time lag $\theta$ is the t intercept and D is the diffusion coefficient.

The diffusion coefficient is an important parameter for a variety of reasons. By determining the diffusion coefficient of various polymer and analyte combinations, it is possible to design a very efficient sensor system. Thus, the design and efficiency of polymer based sensors depend in part on the diffusion coefficient. Moreover, the optimum number and kind of polymers in the sensor array depends in part on the diffusion coefficient. Thus, if the analyte is an aromatic polar analyte, certain polymers are better than others. Using the methods of the present invention, optimum polymers can be determined because the polymer /analyte pair can be better matched.

According to the invention, the array of sensors comprise the same polymer, only the predetermined thickness of the polymer is different. In this aspect, the thinnest polymer sensor has the most resistance (*i.e.*, fewer conductive paths to traverse) and the thickest polymer sensor shows the least resistance (*i.e.*, the most conductive paths to traverse). However, the thinnest sensor will show steady-state conditions faster compared to the thickest sensor which reach steady state conditions at a slower pace.

## VI. <u>EXAMPLES</u>

### A. <u>MATERIALS</u>

This example illustrates the use of an array of conductive/non-conductive regions sensors. In this example, carbon black is the conductive region material. The carbon black (Black Pearls 2000) is a furnace black material from Cabot Co. (Billerica, MA). The nonconductive region comprises a nonconductive polymer.

To prepare the detector substrates, two parallel bands of gold, 50-100 nm thick and separated by 5 mm, are deposited onto conventional 7.5 cm x 2.5 cm glass slides. The slides are then cut into strips to produce 0.7 cm x 2.5 cm pieces of glass, with each strip of glass having one pair of Au leads spaced 5 mm apart.

The detectors are made from a solution of polymer into which carbon black has been suspended. In this example, 125 mg of the polymer is dissolved in 10 mL of tetrahydrofuran, and carbon black (42 mg) is then suspended in this solution, to produce a composition of 75% polymer and 25% carbon black by weight of solids.

One coating of this suspension is applied to each substrate yielding a film thickness of $\approx$ 1 micron as can be determined by atomic force microscopy. For larger sensor thicknesses, more coats are used.

### B. <u>METHODS</u>

The dc resistance of each detector is determined as a function of time using a simple two-point resistance configuration. Contacts are made to the gold lines by pressure-contacting electrical leads using flat-jawed alligator clips. Resistance data is acquired using a Hydra 2620A Data Acquisition Unit (John Fluke Mfg. Co.; Everett, WA) which is interfaced to a personal computer. All of the films had resistance values below the 10 M$\Omega$ limit of the Hydra 2620A.

To initiate an experiment, five copies of a given detector type are placed into the glass chamber and a background flow of nitrogen is introduced until the resistance of the detectors is stabilized. Solvent vapor streams are then passed over the detectors. The background and analyte flow rates are monitored using two flow meters (Gilmont Instruments, Inc.) which have limits of 0.2 L min$^{-1}$ to 15.0 L min$^{-1}$ and 0.0015 L min$^{-1}$ to 0.310 L min$^{-1}$, respectively. In a typical experiment, resistance data on the detectors are collected for 150 s with just the background gas flowing (typically about 1 - 2 L min$^{-1}$) to serve as a baseline. This is followed by a 10 s to 2 minutes data collection while the detectors are exposed to the analyte vapor stream (typically about 200 - 300 mL min$^{-1}$). The detectors are then given 200 - 300 s to recover during which pure background gas was passed through the chamber. The exposure times varied, but steady state values of resistance change are always reached for any given exposure time. Resistances for all detectors in a given trial were monitored contemporaneously through the use of the multiplexing capabilities of the Hydra voltmeter. Results are obtained by running two trials of five exposures each, with the trials performed on different days. Each analyte is exposed to five copies of the detector simultaneously and the results.

**Example 1**

This example illustrates a response of an array of sensors having predetermined polymer thicknesses of 100 nm and 500 nm to an analyte.

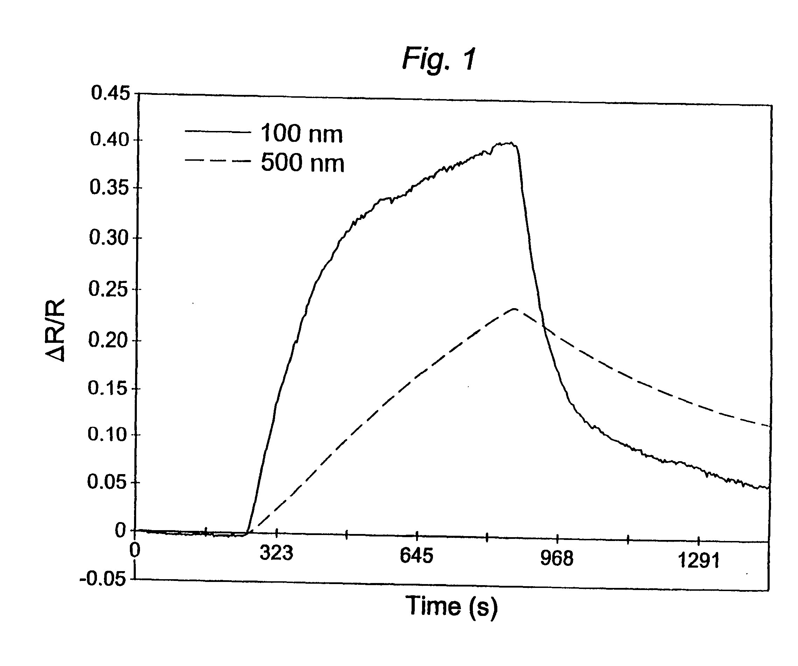

With reference to Figure 1, a response of an array of detectors having predetermined polymer thicknesses of 100 nm and 500 nm to an analyte is illustrated. In this example, the polymer used was polycaprolactone and the analyte used was benzophenone. All the detectors displayed an increase in resistance upon exposure to the vapor, and returned to their baseline values after the vapor was removed. The change in resistance for the thinnest polymer showed steady-state conditions faster compared to the thickest sensor which reach steady-state conditions at a slower pace. The responses are analyzed by calculating the maximum differential response value, $\Delta R_{max}$, observed during the exposure period and dividing it by the baseline value of the resistance, $R_i$, (taken as the resistance value just before the exposure began) and expressed as:

$$\Delta R_{max}/R_i$$

Equation III

A plot of the response flux as a function of time can be used to calculate a diffusion coefficient. In order to measure the diffusivity experimentally in a polymer based sensor, the time lag procedure is used. The time lag $\theta$ is estimated from the time axis intercept and the diffusion coefficient is obtained using Equation II.

**Example 2**

This example illustrates a response of an array of detectors having predetermined polymer thicknesses and analytes having different volatilities.

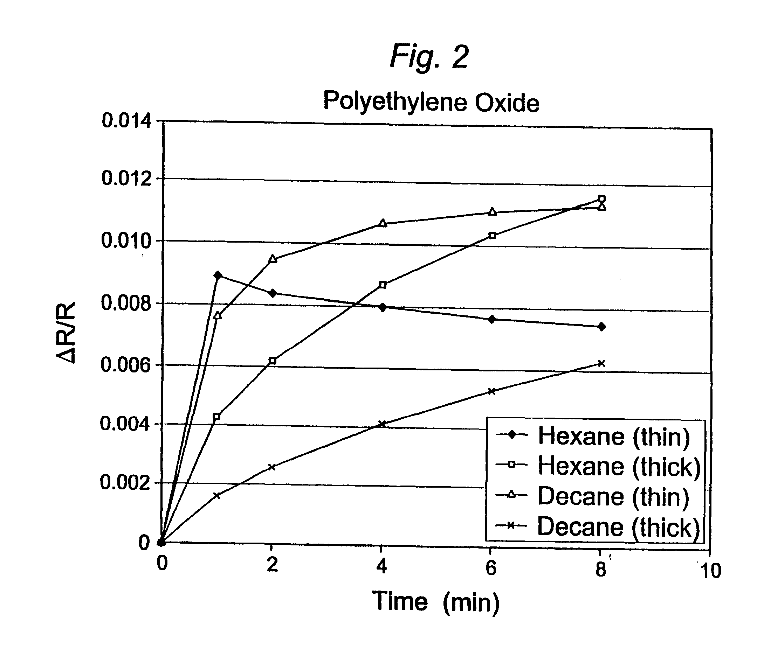

With reference to Figure 2, the responses of an array of detectors having predetermined polymer thicknesses (one thin, one thick) and to different analytes is illustrated. The polymer used was polyethylene oxide and the analytes were hexane and decane. The results indicate that the thin sensor shows steady-state conditions faster compared to the thick sensor which reaches steady state conditions at a slower pace. In addition, the analyte that is more volatile, (hexane), reaches steady-state conditions faster than an analyte that is less volatile (decane). Table 1 shows the responses of the thin and thick sensors versus time as well as the responses of two different analytes.

**TABLE 1**

| Time (min) | 1 | 2 | 4 | 6 | 8 |
|---|---|---|---|---|---|
| Hexane (thin) | 0.008917 | 0.008389 | 0.007985 | 0.007678 | 0.007468 |
| Hexane (thick) | 0.00428 | 0.0062 | 0.008713 | 0.010352 | 0.011586 |
| Decane (thin) | 0.007624 | 0.009453 | 0.010664 | 0.011088 | 0.011286 |
| Decane (thick) | 0.001577 | 0.002578 | 0.004103 | 0.00529 | 0.006279 |

By monitoring the temporal response lag between different film thicknesses to a response, sensors having advantageous features are realized. For example, when monitoring certain volitilities components, the lag time is an important component which can be monitored. False rates can be minimized if the lag profile does not correspond to the expected lag profile.

**Claims**

1. A sensor device comprising a sensor array for detecting an analyte in a fluid, and a detector capable of detecting an electrical response operatively associated with said sensor array;

**characterised in that**:

said sensor array comprises:
a first sensor having a first predetermined polymer thickness; and a second sensor having a second predetermined polymer thickness;
wherein
the polymer of the first and second sensors is the same;
said first predetermined polymer thickness is different than said second predetermined polymer thickness; and said first sensor has a first sensor thickness of 0.01 $\mu$m to 20 $\mu$m.

2. A device as claimed in claim 1 wherein said sensor array has a first sensor thickness of 0.05 $\mu$m to 2 $\mu$m.

3. A device as claimed in claims 1 or 2 , wherein said detector is optimised to detect a member selected from the group consisting of, resistance, capacitance or impedance.

4. A device as claimed in any preceding claim, wherein a sensor in the sensor array comprises a non-conductive polymer mixed with an electrically conductive material.

5. A device as claimed in claim 4 wherein the electrically conductive material is carbon black.

6. A device as claimed in any of claims 1 to 3, wherein said first sensor polymer is a conducting polymer.

7. The use of a device as claimed in any of claims 1 to 6 for measuring a diffusion coefficient of an analyte, said method comprising;

contacting the first sensor with said analyte to elicit a first electrical response;
contacting the second sensor with said analyte to elicit a second electrical response; and
comparing the first response to the second response to calculate a time lag and thereafter measuring the diffusion coefficient of said analyte.

8. The use of a device as claimed in any of claims 1 to 6 for simultaneously determining a partition coefficient and a diffusion coefficient of an analyte, said method comprising:

contacting the first sensor with said analyte to elicit a first electrical response;
contacting the second sensor with said analyte to elicit a second electrical response; and
comparing the first response to the second response thereby simultaneously determining said partition coefficient and said diffusion coefficient of an analyte.

9. The use as claimed in claim 8, wherein said diffusion coefficient is determined using the lag time method.

**Patentansprüche**

1. Sensoreinrichtung mit einem Sensorarray zur Feststellung eines Analyten in einer Flüssigkeit, und einem Detektor, der in der Lage ist, eine elektrische Antwort zu erfassen und der mit dem Sensorarray gekoppelt ist, **<u>dadurch kennzeichnet, dass</u>** das Sensorarray folgendes enthält: einen ersten Sensor mit einer ersten bestimmten Polymerdicke; und einen zweiten Sensor mit einer zweiten bestimmten Polymerdicke; wobei das Polymer des ersten und zweiten Sensors das Gleiche ist; wobei die erste bestimmte Polymerdicke von der zweiten bestimmten Polymerdicke verschieden ist; und der erste Sensor eine erste Sensordicke von 0,01 $\mu$m bis 20 $\mu$m aufweist.

2. Einrichtung nach Anspruch 1, bei der das Sensorarray eine erste Sensordicke von 0,05 $\mu$m bis 2 $\mu$m aufweist.

3. Einrichtung nach Anspruch 1 oder 2, bei der der Detektor so optimiert ist, dass er ein Element aus der Gruppe erfasst, die Widerstand, Kapazität oder Impedanz umfasst.

4. Einrichtung nach einem der vorhergehenden Ansprüche, bei der ein Sensor in dem Sensorarray ein nichtleitendes Polymer enthält, das mit einem elektrisch leitenden Polymer gemischt ist.

**5.** Einrichtung nach Anspruch 4, bei der das elektrisch leitende Material Ruß ist.

**6.** Einrichtung nach einem der Ansprüche 1 bis 3, bei der das erste Sensorpolymer ein leitendes Polymer ist.

**7.** Verwendung einer Einrichtung nach einem der Ansprüche 1 bis 6 zur Messung eines Diffusionskoeffizienten eines Analyten, wobei das Verfahren folgendes umfasst: Kontaktieren des Analyten mit dem ersten Sensor, um eine erste elektrische Antwort hervorzurufen; Kontaktieren des Analyten mit dem zweiten Sensor, um eine zweite elektrische Antwort hervorzurufen; und Vergleichen der ersten Antwort mit der zweiten Antwort, um einen Zeitabstand zu erreichen und danach den Diffusionskoeffizienten des Analyten zu messen.

**8.** Verwendung einer Einrichtung nach einem der Ansprüche 1 bis 6 zur gleichzeitigen Bestimmung eines Verteilungskoeffizienten und eines Diffusionskoeffizienten eines Analyten, wobei das Verfahren umfasst: Kontaktieren des Analyten mit dem ersten Sensor, um eine erste elektrische Antwort hervorzurufen; Kontaktieren des Analyten mit dem zweiten Sensor, um eine zweite elektrische Antowort hervorzurufen; und Vergleichen der ersten Antwort mit der zweiten Antwort, um damit gleichzeitig den Verteilungskoeffizienten und den Diffusionskoeffizienten eines Analyten zu bestimmen.

**9.** Verwendung nach Anspruch 8, bei der der Diffusionskoeffizient unter Verwendung des Zeitabstandsverfahrens bestimmt wird.

**Revendications**

**1.** Dispositif de capteur comprenant un réseau de capteurs pour détecter un analyte dans un fluide, et un détecteur capable de détecter une réponse électrique associée de manière fonctionnelle audit réseau de capteurs ;

   **caractérisé en ce que** :

      ledit réseau de capteurs comprend :
      un premier capteur ayant une première épaisseur de polymère prédéterminée ; et
      un deuxième capteur ayant une deuxième épaisseur de polymère prédéterminée;
      où le polymère des premier et deuxième capteurs est le même ;
      ladite première épaisseur de polymère prédéterminée est différente de ladite deuxième épaisseur de polymère prédéterminée ; et
      ledit premier capteur possède une première épaisseur de capteur de 0,01 $\mu$m à 20 $\mu$m.

**2.** Dispositif selon la revendication 1, où ledit réseau de capteurs possède une première épaisseur de capteur de 0,05 $\mu$m à 20 $\mu$m.

**3.** Dispositif selon la revendication 1 ou 2, où ledit détecteur est optimisé pour détecter un élément choisi parmi le groupe consistant en une résistance, une capacité ou une impédance.

**4.** Dispositif selon l'une quelconque des revendications précédentes, où un capteur dans le réseau de capteurs comprend un polymère non conducteur mélangé à un matériau électriquement conducteur.

**5.** Dispositif selon la revendication 4, où le matériau électriquement conducteur est du noir de carbone.

**6.** Dispositif selon l'une quelconque des revendications 1 à 3, où ledit premier polymère de capteur est un polymère conducteur.

**7.** Utilisation d'un dispositif selon l'une quelconque des revendications 1 à 6, pour mesurer un coefficient de diffusion d'un analyte, ledit procédé consistant à :

      mettre en contact le premier capteur avec ledit analyte pour obtenir une première réponse électrique ;
      mettre en contact et le deuxième capteur avec ledit analyte pour obtenir une deuxième réponse électrique ; et
      comparer la première réponse à la deuxième réponse pour calculer un décalage de temps et mesurer ensuite le coefficient de diffusion dudit analyte.

8. Utilisation d'un dispositif selon la revendication selon l'une quelconque des revendications 1 à 6 pour déterminer simultanément un coefficient de partage et un coefficient de diffusion d'un analyte, ledit procédé consistant à :

mettre en contact le premier capteur avec ledit analyte pour obtenir une première réponse électrique ;
mettre en contact et le deuxième capteur avec ledit analyte pour obtenir une deuxième réponse électrique ; et
comparer la première réponse à la deuxième réponse en déterminant ainsi simultanément ledit coefficient de partage et ledit coefficient de diffusion d'un analyte.

9. Utilisation selon la revendication 8, où ledit coefficient de diffusion est déterminé en utilisant le procédé de temps de décalage.

Fig. 1

EP 1 151 272 B1

*Fig. 2*

Polyethylene Oxide

EP 1 151 272 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10867498 P
- US 10891598 P
- US 5571401 A, Lewis
- US 5675070 A, Gelperin
- US 5697326 A, Mottram
- US 5788833 A, Lewis
- US 5807701 A, Payne
- US 5891398 A, Lewis
- WO 9908105 A
- WO 9927357 A
- US 5945060 A, Buehler
- US 09338339 B
- WO 9900663 A
- WO 9931494 A
- WO 9829563 A
- WO 9839470 A
- WO 9909407 A
- WO 9909408 A

**Non-patent literature cited in the description**

- **Lundström et al.** *Nature,* 1991, vol. 352, 47-50
- **Shurmer ; Gardner.** *Sens. Actuators B,* 1992, vol. 8, 1-11
- **Barker et al.** *Sens. Actuators B,* 1994, vol. 17, 143
- **Gardner et al.** *Sens. Actuators B,* 1994, vol. 18, 240
- **Grate et al.** *Anal. Chem.,* 1995, vol. 67, 2162
- **Grate et al.** *Anal. Chem.,* 1993, vol. 65, A987
- **Grate et al.** *Anal. Chem.,* 1993, vol. 65, A940
- **Hughes et al.** *J. Biochem. and Biotechnol.,* 1993, vol. 41, 77
- **Chang et al.** *Anal. Chim. Acta,* 1991, vol. 249, 323
- **Walt et al.** *Anal. Chem.,* 1996, vol. 68, 2191
- **Gardner et al.** *IEE Proc., Circuits Devices Syst.,* 1995, vol. 142, 321-33
- **K. Buhlmann et al.** *Sensors and Actuators B,* 1998, vol. 49, 156-165
- **Brust, M. ; Walker, M. ; Bethell, D. ; Schiffrin, D.J. ; Whyman, R.** *J. Chem. Soc., Chem. Commun.,* 1994, 801-802
- **Templeton et al.** *J. Am. Chem. Soc.,* 1998, vol. 120, 1906-1911
- **Lee et al.** *Isr. J. Chem.,* 1997, vol. 37, 213-223
- **Hostetler et al.** *LANGMUIR,* 1998, vol. 14, 17-30
- **Ingram et al.** *J. Am. Chem. Soc.,* 1997, vol. 119, 9175-9178
- **Hostetler et al.** *J. Am Chem. Soc.,* 1996, vol. 118, 4212-4213
- **Henglein.** *J. Phys. Chem.,* 1993, vol. 97, 5457-5471
- **Zeiri.** *J. Phys. Chem.,* 1992, vol. 96, 5908-5917
- **Leff et al.** *LANGMUIR,* 1996, 4723-4730
- **R. Andrew McGill et al.** *IEEE Transactions on Ultrasonics, Ferroelectrics, and Frequency Control,* 1998, vol. 45, 1370-1380